(19) **Europäisches Patentamt**
**European Patent Office**
**Office européen des brevets**

(11) **EP 4 566 516 A1**

(12) **EUROPEAN PATENT APPLICATION**

(43) Date of publication:
**11.06.2025 Bulletin 2025/24**

(21) Application number: **24217633.7**

(22) Date of filing: **05.12.2024**

(51) International Patent Classification (IPC):
**A61B 5/00** *(2006.01)* **A61B 18/14** *(2006.01)*
**A61B 18/12** *(2006.01)* **A61B 5/0538** *(2021.01)*

(52) Cooperative Patent Classification (CPC):
**A61B 5/6858; A61B 5/0538; A61B 5/6843;**
**A61B 5/6859; A61B 5/6885; A61B 5/7264;**
A61B 18/1492; A61B 2018/0016; A61B 2018/00267;
A61B 2018/00357; A61B 2018/00577;
A61B 2018/00613; A61B 2018/00875

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **06.12.2023 US 202318530267**

(71) Applicant: **Biosense Webster (Israel) Ltd.**
**2066717 Yokneam (IL)**

(72) Inventors:
• **GOVARI, Assaf**
**2066717 Yokneam (IL)**
• **ALTMANN, Andres Claudio**
**2066717 Yokneam (IL)**
• **GLINER, Vadim**
**2066717 Yokneam (IL)**

(74) Representative: **Carpmaels & Ransford LLP**
**One Southampton Row**
**London WC1B 5HA (GB)**

(54) **IMPROVED ESTIMATION OF CATHETER PROXIMITY TO TISSUE USING CONTACT FORCE SENSING**

(57) A method to find tissue proximity indications includes inserting a shaft of catheter into body part of living subject, the catheter including an expandable distal-end assembly coupled to a distal end of the shaft that has multiple electrodes disposed thereon. Impedances are measured between each of the electrodes and a reference electrode. Based on measured impedances, subset of the electrodes is identified, that physically contact tissue of the body part. Signals are received from assembly of coils coupled at least one of the distal-end assembly and the distal end of the shaft. Based on signals, estimated is a total contact force exerted on the tissue by the assembly. Based on the identified subset of the electrodes and the estimated total contact force, one or more qualities are inferred, of physical contact between respective electrodes and the tissue. One or more of the inferred qualities of physical contact are output.

**EP 4 566 516 A1**

**Description**

**FIELD OF THE DISCLOSURE**

The present disclosure relates generally to invasive medical probes, and particularly to estimation of proximity of catheters to tissue.

**BACKGROUND OF THE DISCLOSURE**

Techniques to estimate the proximity of an electrode of a catheter to tissue were previously proposed in the patent literature. For example, U.S. Patent Application Publication 2022/0183748 describes a method for finding tissue proximity indications that includes inserting a catheter into a body part of a living subject such that electrodes of the catheter contact tissue at respective locations within the body part. Signals are received that were provided by the electrodes. The method further includes selectively rewarding and penalizing a reinforcement learning agent over reinforcement learning exploration phases to learn at least one tissue proximity policy responsively to at least one of the received signals. The method additionally includes applying the reinforcement learning agent in reinforcement learning exploitation phases to find respective tissue-proximity actions to be taken that maximize respective expected rewards responsively to the at least one tissue proximity policy. The method also includes providing respective derived tissue-proximity indications of proximity of a given one of the electrodes with the tissue responsively to the found respective tissue-proximity actions.

The present disclosure will be more fully understood from the following detailed description of the examples thereof, taken together with the drawings, in which:

**BRIEF DESCRIPTION OF THE DRAWINGS**

Fig. 1 is a schematic, pictorial illustration of a catheter-based electro-anatomical (EA) mapping and ablation system, in accordance with an example of the present disclosure;

Fig. 2 is a schematic, pictorial illustration of a basket assembly configured to electrically sense tissue proximity and magnetically acquire indication of contact force, in accordance with examples of the present disclosure;

Fig. 3 is a flow chart that schematically illustrates a method to infer electrode touch quality for the basket cage of Fig. 2, in accordance with an example of the present disclosure.

Fig. 4 is a schematic graph of statistically inferred electrode's touch quality level using estimated contact force, in accordance with examples of the present disclosure; and

Fig. 5 is a flow chart that schematically illustrates a method to infer the touch quality of an assembly electrode with tissue, in accordance with an example of the present disclosure.

**DETAILED DESCRIPTION OF EXAMPLES**

OVERVIEW

A wall tissue of a cavity of an organ of a patient, such as a cardiac chamber, can be mapped and/or ablated using a catheter having multiple electrodes fitted at an expandable distal-end assembly of the catheter. In a mapping and/or ablation procedure in a cardiac chamber, the physician expands the assembly and manipulates the expanded distal-end assembly for the electrodes to contact chamber walls to acquire or apply electrical signals.

The quality of electrical mapping and/or ablation depends on the quality of physical contact of the electrodes with wall tissue (also called in the disclosure "touch quality, (TQ)"). The quality scale may use arbitrary units (e.g., numbers from 0 to 10) or can be given in physical units of contact force (e.g., from 0 to 10 Dynes in steps of 1 Dyne (=1 gram·cm·sec$^{-2}$)).

One way to estimate the contact force with tissue of an entire assembly (e.g., not of a specific electrode) is using a local transmitter-receiver mode of proximal and distal electromagnetic coils (EMCs), where the distal EMC is disposed on a distal portion of the expandable assembly and the proximal EMC is disposed on a distal end of a shaft of the catheter. This local transmitter-receiver mode yields an accurate estimation of assembly's deflection, $\Delta l$ in 3D relative to the distal end of the catheter's shaft. In one example, the technique employs 3 coils distributed on the assembly's distal ends in 3 different XYZ orientations (e.g., on 3 different splines in the case of an expandable basket assembly), which enables to sense assembly's 3D position and orientation (e.g., of the basket assembly of the catheter) relative to the distal end of the catheter's shaft. Using the deflection $\Delta l$ (e.g., a change in 3D orientation) together with the known spring constant of the distal end assembly (the elastic cage) a processor can calculate the contact force using the spring equation (F=K·$\Delta l$). The spring constant is known from the elastic model of the cage or is known based on lab calibration between known applied forces on the cage and respectively measured cage deflections (e.g., to measure the spring constant). A detailed description of the contact force estimation method for a multi-electrode catheter using location sensors in a local transceiver mode is given in a U.S. Patent Application 18/373,308, filed on September 27, 2023, and titled "Estimation of Contact Force of Catheter Expandable Assembly," which is assigned to the assignee of the current patent application.

The electrical impedance of an electrode of a catheter is considered an electrode-specific measure of how close the electrode is to a wall tissue. As the range of impedances encountered by the electrodes is patient and procedure specific, the disclosed algorithm is used to continuously monitor each electrode impedance and look for changes. The impedance measurements may be normalized by detecting the lowest and highest values recorded over time. Lower impedances are typically measured for electrodes well within the blood pool of a cardiac chamber, and higher impedance occurs when the electrode is in close proximity, or physical contact (e.g., above 1 Dyne) with tissue.

For example, touch proximity (TP) can be estimated this way by measuring the electrical impedance between electrodes disposed over the distal assembly and the body tissue and patch electrodes. Examples of such an electrical technique are further described in U.S. Patent Application Publication 2022/0401032. TPI can also be measured more locally between electrodes disposed over the distal assembly and a reference electrode at the distal end of the shaft or on the distal assembly.

Examples of the present disclosure that are described herein provide a technique in which a processor infers a real-time quality of physical contact between an electrode and tissue based on measured impedances related to contact forces applied on the distal end assembly. The technique achieves an improved estimation of touch quality, by determining a relation between the contact force applied by the to tissue the distal end assembly as a whole and impedance measured by the electrodes. The relation is applicable to any electrode despite measuring the contact force of the entire assembly only.

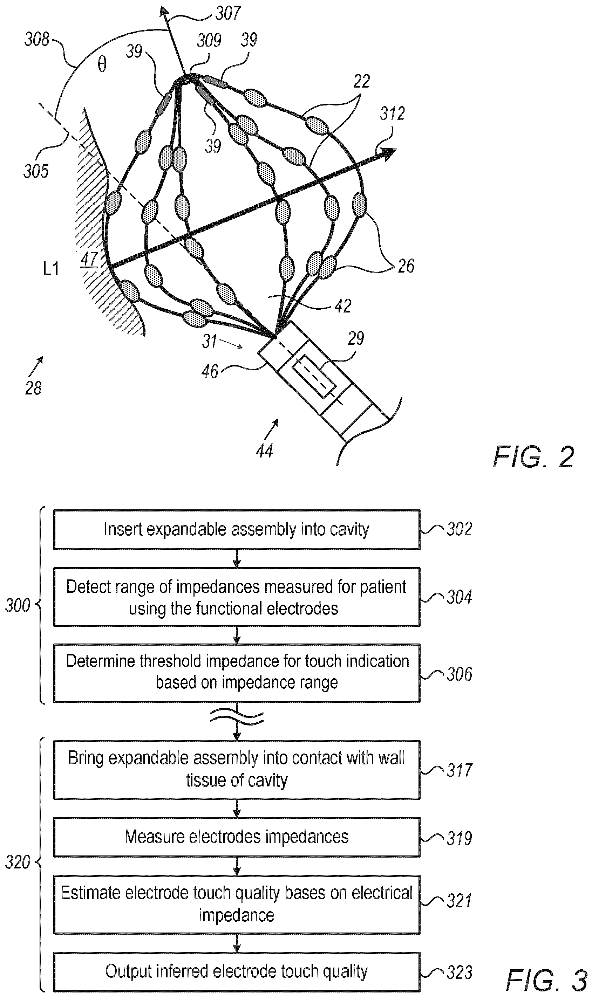

To achieve the relation, a processor running the disclosed technique identifies a subset of the assembly's electrodes that are indicated to be physically contacting the tissue wall by (i) identifying a region over the assembly surface (e.g., over an approximate sphere in case of a basket assembly) that is contact with tissue, meaning region largely centered about a direction orthogonal to the assembly's deflection direction (as seen in Fig. 2), and (ii) considering in that region only electrodes with sufficient impedance to indicate these are in firm enough contact with tissue (e.g., electrodes with impedances above a given threshold).

The processor applies an analytical model, described in Fig. 2, to the identified subset of electrodes to obtain a relation for any electrode between its measured impedance and its underlying contact force with tissue.

Thus, during a clinical process, such as ablation, the processor can estimate in real-time from the measured impedance and the detected force applied on the distal end assembly, the contact force of individual electrodes of the expandable distal-end assembly to determine, for example, if the electrode TQ is sufficient for applying ablation energy.

In another example, a processor can statistically infer the TQ of individual electrodes using Bayesian estimation (described in Fig. 3) to evaluate the most likely touch quality value on a given electrode. The indicated contact force is the input, and the most likely touch quality (e.g., in terms of contact force) is the inferred output. Bayesian estimation may change the initial (electrical only) estimation of the most likely contact force, as described in Fig. 3.

Yet another way to statistically infer the quality of the physical contact is to use a neural network trained with the electrical signals and respectively estimated contact forces, or directly by using the acquired magnetic position data for the training.

## SYSTEM DESCRIPTION

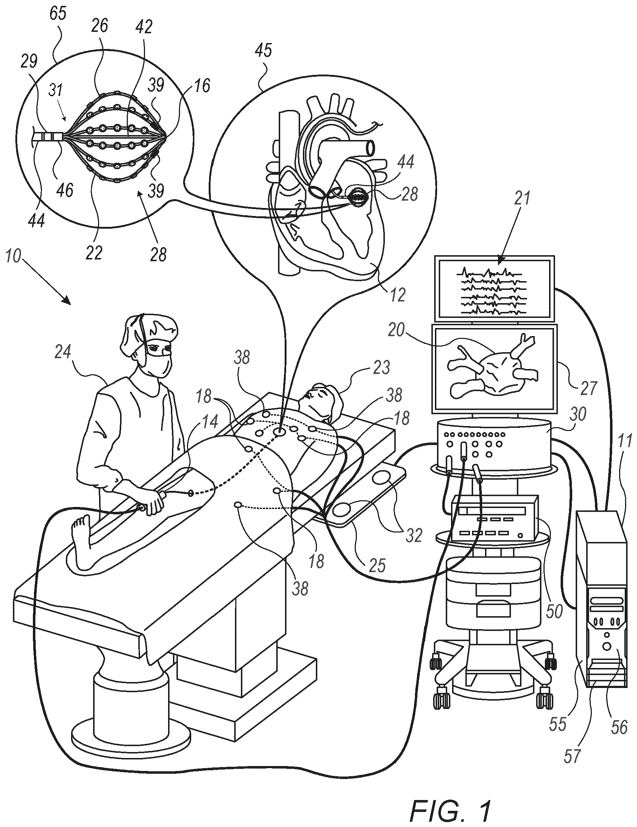

Fig. 1 is a schematic, pictorial illustration of a catheter-based electroanatomical (EA) mapping and ablation system 10, in accordance with an example of the present disclosure.

System 10 includes one or more catheters, which are percutaneously inserted by physician 24 through the patient's vascular system into a chamber or vascular structure of a heart 12. Typically, a delivery sheath catheter is inserted into the left or right atrium near a desired location in heart 12. Thereafter, one or more catheters can, in turn, be inserted into the delivery sheath catheter to arrive at the desired location. The one or more catheters may include catheters dedicated to sensing intracardiac electrogram (IEGM) signals, catheters dedicated to ablating and/or catheters dedicated to both sensing and ablating. An example basket catheter 14 that is configured for ablation sensing IEGM is illustrated herein. As another example, catheter 14 can be a tip catheter for ablation and sensing fitted with a contact force mechanism described in U.S. Patent 8,535,308, which is assigned to the assignee of the current application.

As seen in inset 45, physician 24 brings a basket assembly 28 (also called hereinafter "expandable distal-end assembly 28") fitted on a shaft 44 of catheter 14 into contact with the heart wall for sensing a target site in heart 12. For ablation, physician 24 similarly brings a distal end of an ablation catheter to a target site for ablating.

As seen in inset 65, basket catheter 14 is an exemplary catheter that includes one and preferably multiple electrodes 26 optionally distributed over a plurality of splines 22 at expandable distal-end assembly 28 and configured to ablate and/or sense IEGM signals. Catheter 14 additionally includes (i) a proximal position sensor 29 (e.g., dual axial sensor (DAS) 29 with two orthogonal EMCs or tri-axial sensor (TAS) comprising three orthogonal EMCs) embedded in a distal end 46 of shaft 44 near basket assembly 28, and (ii) three distal position sensors 39 (e.g., single axial sensor (SAS) 39 comprising a single EMC) for a tracking position of the distal end of basket assembly 28. Optionally and preferably, position sensors 29 and 39 are magnetic-based position sensors that include magnetic coils for sensing three-dimensional (3D) position. An example reference electrode 31 located at the base of assembly 28 is also seen.

Fig. 2 below shows how the EMCs of distal sensors 39 and of proximal sensor 29 are used in a transmitter-receiver mode for estimating contact force of an elastic basket cage with tissue. At the same time, impedance signals between electrodes 26 (bipolar signals) and/or between electrodes 26 and electrode patches 38 (unipolar signals) are used for electrically estimating tissue proximity.

As described in Figs. 2 and 3, the disclosed technique quantifies the quality of contact (i.e., the TP) of each of a plurality of electrodes 26 with the tissue wall based on the impedance detection related by the model described in Fig. 2 to the contact force detection.

Magnetic position sensors (29, 39) (i.e., an assembly of coils) may also be operated together with an external location pad 25 that includes a plurality of magnetic coils 32 configured to generate magnetic fields in a predefined working volume. To prevent confusion among signals, the frequencies of these fields are different from any given frequency used in the local transmitter-receiver mode for contact force detection. Real-time orientation of basket assembly 28 of catheter 14 can, in this way, be calculated from tracked locations of sensors 29 and 39 (locations being tracked using magnetic fields generated with location pad 25 and sensed by magnetic-based position sensors 29 and 39). This relative orientation is manifested by an angle formed between a distal end 46 and a longitudinal axis 42 of expandable assembly 28 (to a distal edge 16 of the assembly).

Details of the magnetic-based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25, as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more electrodes at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses that may be used to effect irreversible electroporation (IRE), or combinations thereof.

Patient interface unit (PIU) 30 is configured to establish electrical communication between catheters, electrophysiological equipment, power supply, and a workstation 55 for controlling operation of system 10. Electrophysiological equipment of system 10 may include, for example, multiple catheters, location pad 25, body surface ECG electrodes 18, electrode patches 38, ablation energy generator 50, and recorder 11. Optionally and preferably, PIU 30 additionally includes processing capability for implementing real-time computations of catheter locations and for performing ECG calculations.

Workstation 55 includes memory 57, processor unit 56 with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded electrograms 21 in representative visual indicia or imagery superimposed on the rendered anatomical map 20, (iii) displaying real-time location and orientation of multiple catheters within the heart chamber, and (iv) displaying on display device 27 sites of interest such as places where ablation energy has been applied. One commercial product embodying elements of the system 10 is available as the CARTO™ 3 System, available from Biosense Webster, Inc., 31A Technology Drive, Irvine, CA 92618.

While Fig. 1 describes a basket assembly, the figure and disclosed method are applicable, *mutatis mutandis,* to a multi-ray assembly and a balloon assembly.

ESTIMATION OF TOUCH QUALITY (TQ) OF AN ELECTRODE OF EXPANDABLE ASSEMBLY

Fig. 2 is a schematic, pictorial illustration of the catheter expandable distal end assembly 28 in contact with tissue 47, in accordance with examples of the present disclosure.

As seen, expandable distal end assembly 28 develops an angle Θ 308 with respect to the distal end 46 of shaft 44. Angle 308 is defined between a longitudinal axis 307 of the expandable distal end assembly and a longitudinal axis 305 of the distal end 46. Using EMCs 39, EMCs of TAS 29, and known relations between a distal edge 309 of the assembly and distal end 46, the processor can readily calculate angle Θ 308. The processor can calculate the amount of deflection $\varDelta l$ of distal edge 309 based on the tilt angle and the length of the expandable distal end assembly (e.g., length 241). Using the deflection $\varDelta l$ (e.g., a change in 3D orientation) together with the known spring constant of the distal end assembly (the elastic cage) a processor can calculate the contact force using the spring equation ($F=K \cdot \varDelta l$).

The processor calculates a direction 312 in space (orthogonal to the direction of longitudinal axis 307) that is pointing away from the area of tissue contact. Electrodes 314, $e_1$, $e_2$, ... $e_k$, in this area, which give respective impedance readings $z_1$, $z_2$, ... $z_k$ above a threshold (e.g., 200 Ohms), are identified. Based on the known magnitude and direction of the contact force and the identified electrodes with respective impedance readings above the threshold, the force distribution over the identified electrodes may be determined.

Impedance reading can be between any electrode 314 and electrode patches 38 (unipolar signals), the patches used as reference, or between any electrode 314 and the reference electrode 31 on the distal end assembly. Typically, the threshold impedance is determined per patient during the procedure (e.g., by detecting the lowest and highest impedance values recorded over time.

The processor may relate impedance to contact force by considering the total impedance,

$$Z_T = \sum_{j=1}^{k} z_j$$

, to represent the measured total contact force, assumed to be the result of contact of the electrodes of the subset, $f_T = \sum_{j=1}^{k} f_j$, i.e., exerted on tissue by the subset of electrodes with some relation factor, $c$, $Z_T = cf_T$. Typical value of c can be 30, if for example, a total impedance of 900 Ohms per is measured at a total contact force of 30 Dynes. Given any electrode impedance its contact force can be estimated by dividing its impedance by the factor c. For example, the estimated contact force of an electrode of 100 Ohms is 0.3 Dynes, indicating the electrode is in a blood pool. On the other hand, the estimated contact force of an electrode of 240 Ohms is 8 Dynes, indicating the electrode has a good TQ with tissue, and therefore can be used in ablation.

A METHOD FOR ESTIMATING OF TOUCH QUALITY (TQ) OF AN ELECTRODE OF EXPANDABLE ASSEMBLY

Fig. 3 is a flow chart that schematically illustrates a method to infer electrode touch quality for the basket cage 28 of Fig. 2, in accordance with an example of the present disclosure. The method includes two phases: An impedance to force relating phase 300 at the beginning of a clinical procedure to identify the threshold impedance used provide touch indication and a subsequent TQ estimation phase 320. The impedance to force relating phase can be repeated during the clinical procedure.

The algorithm, according to the presented example, carries out a process that begins with physician 24 inserting the expandable distal-end assembly into a cavity (e.g., a cardiac chamber), at basket assembly insertion step 302.

Next, impedance values are sampled, and the range of impedance detected for the patient is determined.

At an impedance thresholding step 306, processor 56 defines the threshold impedance for touch indication based on impedance.

In phase 320, at assembly contacting step 317, the physician brings the basket assembly into contact with the cavity wall tissue (e.g., tissue 47) of the patient, to for example ablate tissue.

At impedance measurement step 319, the processor measures the impedances of the electrodes of the basket assembly.

At an electrical estimation of touch quality step 321, processor 56 estimates a touch quality (e.g., the contact force) of the electrode (e.g., electrodes 26) based on electrical impedance signals.

Finally, the processor outputs the inferred electrode touch quality, at outputting step 323. The information may be displayed to the physician and/or used by another algorithm, such as an EA mapping algorithm or an ablation algorithm.

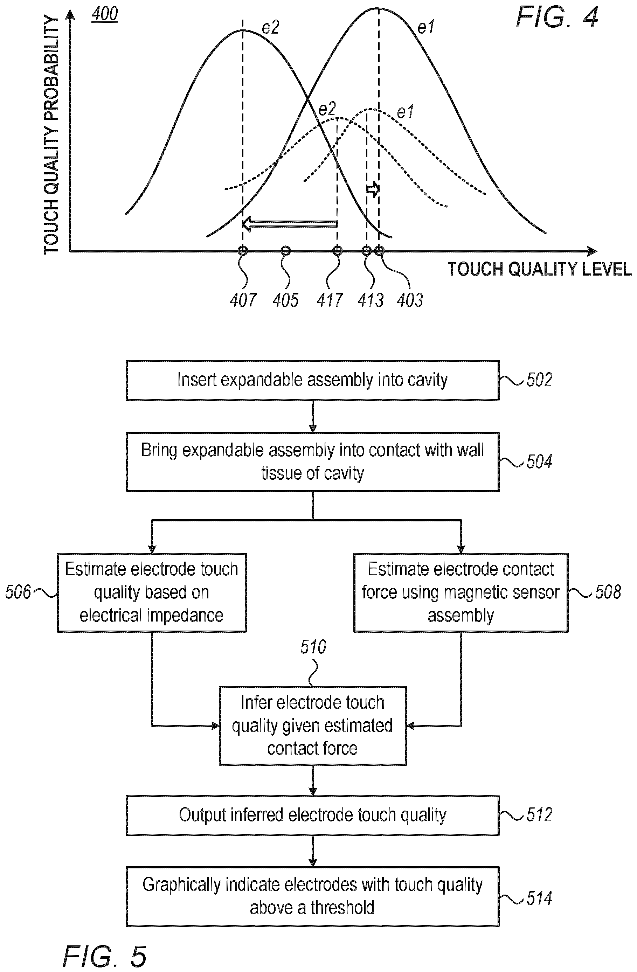

The flow chart shown in Fig. 3 is chosen purely for the sake of conceptual clarity. Other possible steps (such as graphically encoding the TQ, as done in Fig. 5) are omitted from the disclosure herein purposely to provide a more simplified flow chart.

INFERRING ELECTRODE TOUCH QUALITY STATISTICALLY

The inference method described using Fig. 2 is largely an analytical one. A statistical way to infer the touch quality of an electrode from impedance is using a prior estimation of contact force in a Bayesian estimation method. The Bayesian estimation evaluates the most likely value of the contact force on a given electrode. The estimated contact force is the input, and the touch quality is the inferred output, in the form of a statistical distribution with a most likely value.

Fig. 4 is a schematic graph 400 of statistically inferred electrode's touch quality levels using estimated contact force, in accordance with examples of the present disclosure. Fig. 4 shows the probability distribution of touch quality for two different electrodes e1 and e2, such as two of electrodes 26. As seen, the disclosed technique finds the probability of a touch quality, given a measured touch force. For an electrode e1, the most likely touch quality is value 403 which is above the given threshold 405 (e.g., above 1 Dyne). For electrode e2, the most likely touch quality is value 407 which is below threshold 405.

In Fig. 4, the most likely prior touch qualities, 413 and 417 estimations for electrodes e1 and e2, are based solely on electrical impedance. As seen, electrical impedances yield very similar values 413 and 417, with both electrodes considered to have sufficient touch quality. As seen, a processor using the disclosed technique updates the touch quality of electrode e2 as being at an insufficient level 407. In contrast, the prior electrical estimation derived level 417 was shown to be larger than threshold 405.

## A METHOD FOR ESTIMATING CONTACT FORCE OF SPLINE OF CATHETER EXPANDABLE ASSEMBLY

Fig. 5 is a flow chart that schematically illustrates a method to infer the touch quality of an assembly electrode with tissue, in accordance with an example of the present disclosure. The algorithm, according to the presented example, carries out a process that begins with physician 24 inserting the expandable distal-end assembly into a cavity (e.g., a cardiac chamber), at basket assembly insertion step 502.

Next, at assembly contacting step 504, the physician brings the basket assembly into contact with the cavity wall tissue (e.g., tissue 47), which leads to the basket assembly changing shape or orientation relative to the distal end of the shaft.

At an electrical estimation of touch quality step 506, processor 56 estimates a touch quality of an electrode (e.g., an electrode 26) based on electrical impedance signals.

At a magnetic estimation of contact force step 508, processor 56 estimates contact force on the basket using signals from contact force sensor assembly.

Using the contact force estimated in step 508 and the impedance determined in step 506, processor 56 infers the electrode touch quality, at electrode touch quality inferencing step 510.

Finally, the processor outputs the inferred electrode touch quality, at outputting step 512. The information may be used by another algorithm, such as an EA mapping algorithm or an ablation algorithm.

Finally, at a graphics step 514, the processor graphically indicates electrodes 26 that have a touch quality above a given threshold. The information may be displayed to the physician.

The flow chart shown in Fig. 5 is chosen purely for the sake of conceptual clarity. Other possible steps (such as imaging the catheter) are omitted from the disclosure herein purposely to provide a more simplified flow chart.

## EXAMPLES

### Example 1

A method to find tissue proximity indications includes inserting a shaft (44) of a catheter (14) into a body part of a living subject (23), the catheter (14) including an expandable distal-end assembly (28) coupled to a distal end (46) of the shaft (44), the assembly (28) having multiple electrodes (26) disposed thereon. Impedances are measured between each of the electrodes (26) and a reference electrode (31). Based on the measured impedances, a subset of the electrodes (26) is identified, that physically contact tissue of the body part. Signals are received from an assembly of coils (29, 39) coupled at at least one of the distal-end assembly (28) and the distal end (46) of the shaft. Based on the signals, estimated is a total contact force exerted on the tissue by the assembly (28). Based on the identified subset of the electrodes (26) and the estimated total contact force, one or more qualities are inferred, of physical contact between respective electrodes (26) and the tissue. One or more of the inferred qualities of physical contact are output (323).

### Example 2

The method according to example 1, wherein inferring the qualities of physical contact comprises, using the subset of electrodes (26), relating the impedances into contact force per any electrode (26) of the assembly (28).

### Example 3

The method according to any of examples 1 and 2, wherein outputting the qualities of the physical contact comprises providing the qualities of physical contact as numbers on a scale.

### Example 4

The method according to any of examples 1 through 3, and comprising outputting a number when a corresponding estimated electrode's contact force is above a given threshold and the electrode's (26) impedance is within an estimated range of impedances.

### Example 5

The method according to any of examples 1 through 4, wherein inferring a quality among the qualities of an electrode's (26) physical contact comprises using Bayesian statistics to deduce a probability (403, 407) of the physical contact force being above a given threshold (405) contact force.

### Example 6

The method according to any of examples 1 through 4, wherein inferring a quality among the qualities of an electrode's (26) physical contact comprises using a neural network (NN) model to deduce a probability of the quality based on the estimated contact forces.

### Example 7

The method according to any of examples 1 through 6, wherein the expandable distal-end assembly (28) comprises one of multiple spines (22) and multiple rays on which the electrodes (26) are disposed, and wherein estimating the contact forces comprises estimating the contact forces exerted by one of one or more of the splines (22) and one or more of the rays.

Example 8

The method according to any of examples 1 through 7, wherein the expandable distal-end assembly comprises a plurality of splines (22) arranged in one of a basket assembly (28) and a multi-ray assembly.

Example 9

The method according to any of examples 1 through 8, wherein receiving the signals from the assembly of coils (29, 39) comprises using electromagnetic coils (EMC) in a local transmitter-receiver layout of the assembly (28).

Example 10

The method according to any of examples 1 through 9, wherein measuring impedances comprises receiving at least one of bipolar and unipolar signals acquired by the catheter (14).

Example 11

A system (10) to find tissue proximity indications, the system including a catheter (14) and a processor (56). The catheter (14) includes a shaft (44) configured for insertion into a body part of a living subject (23), the catheter further including an expandable distal-end assembly (28) coupled to a distal end (46) of the shaft, the distal-end assembly (28) having multiple electrodes (26) disposed thereon. The processor is configured to (i) measure impedances between each of the electrodes (26) and a reference electrode (31), (ii) based on the measured impedances, identify a subset of the electrodes (26) that physically contact tissue of the body part, (iii) receive signals from an assembly of coils (29, 39) coupled at at least one of the distal-end assembly (28) and the distal end (46) of the shaft, (iv) estimate, based on the signals a total contact force exerted on the tissue by the assembly (28), (v) based on the identified subset of the electrodes (26) and the estimated total contact force, infer one or more qualities of physical contact between respective electrodes (26) and the tissue, and (vi) output one or more of the inferred qualities of physical contact.
Although the examples described herein mainly address cardiac diagnostic applications, the methods and systems described herein can also be used in other medical applications.
It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

**Claims**

1. A system (10) to find tissue proximity indications, the system comprising:

   a catheter (14) comprising a shaft (44) configured for insertion into a body part of a living subject (23), the catheter further comprising an expandable distal-end assembly (28) coupled to a distal end (46) of the shaft, the distal-end assembly (28) having multiple electrodes (26) disposed thereon; and
   a processor (56), which is configured to:

      measure impedances between each of the electrodes (26) and a reference electrode (31);
      based on the measured impedances, identify a subset of the electrodes (26) that physically contact tissue of the body part;
      receive signals from an assembly of coils (29, 39) coupled at at least one of the distal-end assembly (28) and the distal end (46) of the shaft;
      estimate, based on the signals a total contact force exerted on the tissue by the assembly (28);
      based on the identified subset of the electrodes (26) and the estimated total contact force, infer one or more qualities of physical contact between respective electrodes (26) and the tissue; and
      output one or more of the inferred qualities of physical contact.

2. The system according to claim 1, wherein the processor is configured to infer the qualities of physical contact by, using the subset of electrodes, relating the impedances into contact force per any electrode of the assembly.

3. The system according to claim 1 or claim 2, wherein the processor is configured to output the qualities of the physical contact by providing the qualities of physical contact as numbers on a scale.

4. The system according to claim 3, wherein the processor is further configured to output a number when a corresponding estimated electrode's contact force is above a given threshold and the electrode's impedance is within an estimated range of impedances.

5. The system according to any of claims 1 to 4, wherein the processor is configured to infer a quality among the qualities of an electrode's physical contact by using Bayesian statistics to deduce a probability of the physical contact force being above a given threshold contact force.

6. The system according to any of claims 1 to 4, wherein the processor is configured to infer a quality among the qualities of an electrode's physical contact by using a neural network (NN) model to deduce a probability of the quality based on the estimated contact forces.

7. The system according to any preceding claim, wherein the expandable distal-end assembly comprises one of multiple spines and multiple rays on which the electrodes are disposed, and wherein the processor is configured to estimate the contact forces by estimating the contact forces exerted by one of one or more of the splines and one or more of the rays.

8. The system according to any preceding claim, wherein the expandable distal-end assembly comprises a plurality of splines arranged in one of a basket assembly and a multi-ray assembly.

9. The system according to any preceding claim, wherein the processor is configured to receive the signals from the assembly of coils by using electromagnetic coils (EMC) in a local transmitter-receiver layout of the assembly.

10. The system according to any preceding claim, wherein the processor is configured to measure impedances by receiving at least one of bipolar and unipolar signals acquired by the catheter.

FIG. 1

*FIG. 2*

| | |
|---|---|
| Insert expandable assembly into cavity | 302 |
| Detect range of impedances measured for patient using the functional electrodes | 304 |
| Determine threshold impedance for touch indication based on impedance range | 306 |
| Bring expandable assembly into contact with wall tissue of cavity | 317 |
| Measure electrodes impedances | 319 |
| Estimate electrode touch quality bases on electrical impedance | 321 |
| Output inferred electrode touch quality | 323 |

*FIG. 3*

FIG. 4

400

TOUCH QUALITY PROBABILITY

e2    e1

e2    e1

TOUCH QUALITY LEVEL

407  405  417  413  403

---

| Insert expandable assembly into cavity | 502 |

↓

| Bring expandable assembly into contact with wall tissue of cavity | 504 |

↓

506 | Estimate electrode touch quality based on electrical impedance |

| Estimate electrode contact force using magnetic sensor assembly | 508 |

510

| Infer electrode touch quality given estimated contact force |

↓

| Output inferred electrode touch quality | 512 |

↓

| Graphically indicate electrodes with touch quality above a threshold | 514 |

FIG. 5

Europäisches
Patentamt
European
Patent Office
Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/077180 A1 (GOVARI ASSAF [IL] ET AL) 18 March 2021 (2021-03-18)<br>* paragraph [0039] *<br>* paragraph [0056] - paragraph [0059] *<br>* paragraph [0069] - paragraph [0072] *<br>* paragraph [0092] - paragraph [0094] *<br>* paragraph [0114] - paragraph [0119] *<br>* figures 1-9, 13, 14 * | 1-10 | INV.<br>A61B5/00<br>A61B18/14<br>A61B18/12<br>A61B5/0538 |
| A | US 2023/346455 A1 (BEECKLER CHRISTOPHER THOMAS [US] ET AL) 2 November 2023 (2023-11-02)<br>* paragraph [0074] - paragraph [0076]; figure 2 * | 1-10 | |
| A | EP 3 741 298 A2 (BIOSENSE WEBSTER ISRAEL LTD [IL]) 25 November 2020 (2020-11-25)<br>* paragraph [0079] * | 1-10 | |
| A | US 2014/180152 A1 (MASKARA BARUN [US] ET AL) 26 June 2014 (2014-06-26)<br>* paragraph [0050] * | 1-10 | TECHNICAL FIELDS SEARCHED (IPC)<br><br>A61B |

The present search report has been drawn up for all claims

| Place of search | Date of completion of the search | Examiner |
|---|---|---|
| The Hague | 19 February 2025 | Costa Angeli, M |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

EP 4 566 516 A1

## ANNEX TO THE EUROPEAN SEARCH REPORT
## ON EUROPEAN PATENT APPLICATION NO.

EP 24 21 7633

This annex lists the patent family members relating to the patent documents cited in the above-mentioned European search report.
The members are as contained in the European Patent Office EDP file on
The European Patent Office is in no way liable for these particulars which are merely given for the purpose of information.

19-02-2025

| Patent document cited in search report | | Publication date | Patent family member(s) | | Publication date |
|---|---|---|---|---|---|
| US 2021077180 | A1 | 18-03-2021 | CN | 112472275 A | 12-03-2021 |
| | | | EP | 3791816 A2 | 17-03-2021 |
| | | | IL | 277092 A | 25-03-2021 |
| | | | JP | 2021053373 A | 08-04-2021 |
| | | | US | 2021077180 A1 | 18-03-2021 |
| US 2023346455 | A1 | 02-11-2023 | EP | 4268746 A1 | 01-11-2023 |
| | | | JP | 2023164370 A | 10-11-2023 |
| | | | US | 2023346455 A1 | 02-11-2023 |
| EP 3741298 | A2 | 25-11-2020 | CN | 111973272 A | 24-11-2020 |
| | | | EP | 3741298 A2 | 25-11-2020 |
| | | | IL | 274725 A | 30-11-2020 |
| | | | JP | 2020189095 A | 26-11-2020 |
| | | | US | 2020367829 A1 | 26-11-2020 |
| | | | US | 2022401032 A1 | 22-12-2022 |
| US 2014180152 | A1 | 26-06-2014 | CN | 104869889 A | 26-08-2015 |
| | | | EP | 2934288 A1 | 28-10-2015 |
| | | | JP | 6517277 B2 | 22-05-2019 |
| | | | JP | 2016502885 A | 01-02-2016 |
| | | | JP | 2017200598 A | 09-11-2017 |
| | | | US | 2014180152 A1 | 26-06-2014 |
| | | | US | 2016331267 A1 | 17-11-2016 |
| | | | WO | 2014100631 A1 | 26-06-2014 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20220183748
- US 37330823
- US 20220401032
- US 8535308 B
- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B